# EUROPEAN PATENT APPLICATION

(11) **EP 4 696 223 A1**
(43) Date of publication of application: **18.02.2026**
(21) Application number: 24194514.6
(22) Date of filing: 14.08.2024
(51) Int. Cl.: A61B 5/00, A61B 34/20, A61B 34/00

(54) **A CONTROLLER FOR DEPLOYMENT OF MESH DEVICES**

(71) Applicant: Koninklijke Philips N.V., 5656 AG Eindhoven (NL)
(72) Inventor: FOTOUHI, Javad, Eindhoven (NL); LEE, Brian Curtis, Eindhoven (NL); SINHA, Ayushi, 5656AG Eindhoven (NL); FEIZPOUR, Amin, Eindhoven (NL); BALICKI, Marcin Arkadiusz, Eindhoven (NL); PATRICIU, Alexandru, Eindhoven (NL)
(74) Representative: Philips Intellectual Property & Standards

(57) **Abstract**

The present invention relates to a device and method for assisting in placement of an endovascular device for treatment of an aneurism comprising a data processor configured to receive image data representing an anatomical region of interest including a vasculature, and at least a distal portion of a placement device together with the endovascular device located in the vasculature. The data processor or controller is configured to interpret the image data and to determine instructions for positioning and deployment of the endovascular device while the endovascular device is in engagement with an inner shaft of the placement device.

## Description

### FIELD OF THE INVENTION

The present invention relates to minimally invasive and image-guided procedures for deploying stent-like devices for medical therapy. In a particular embodiment, the present invention relates to devices and methods for controlling the deployment of a flow-diverter which can be placed across an aneurysm to redirect blood flow away from the aneurysm.

### BACKGROUND OF THE INVENTION

Stent-like deployment is used to perform a medical therapy of a blood vascular disease or damage (e.g. local aneurysm, narrowed vessels due to clots, calcification..., vascular lesion ...).

For instance, flow diversion is an increasingly popular endovascular method of treating cerebral aneurysms, potentially due to improved aneurysm occlusion with no increase in complication rates. The deployment of the flow diverter device is a complex maneuver that requires a number of multiaxial endovascular device movements to ensure that the flow diverter mesh is properly positioned and achieves a well-apposed placement. This is a high-stakes maneuver because the flow diverter cannot be recaptured once deployed and correcting a poor flow diverter placement is challenging if not impossible, likely requiring placement of a second flow diverter. Additionally, flow diverter devices are costly. For these reasons, physicians, hospitals, and patients are all incentivized to produce successful flow diversion on the first try.

The quality of flow diverter (or any other type of stent-like device) deployment is generally defined by the accuracy of the distal and proximal ends of the implant as well as the apposition of the flow diverter mesh against the parent vessel wall. The purpose of the implant is to place a mesh over the aneurysm neck (or start of any other type of vascular disease or damage) which serves as a bed for endothelialization and eventual occlusion of the aneurysm (or other type of vascular disease or damage). The quality of endothelialization and occlusion depends on the apposition of the mesh to the vessel wall - the mesh should be well apposed in order to fully occlude the aneurysm. The placement quality of the distal and proximal ends of the implant is measured by the coverage of the aneurysm, placement in a location that ensures good anchoring of the device, and placement in a location that avoids occlusion of secondary blood vessels branching off of the parent vessel. In practice, only the distal end's placement can be finely controlled. The proximal end's eventual location is determined partially by the device length and partially by the degree of packing of the mesh during placement.

US 2023/0190225 A1 describes a system including a processor circuit that receives intraluminal images of a body lumen. The processor circuit identifies a stent edge within the intraluminal images and calculates a distance between the stent edge and the vessel wall. The processor circuit additionally compares the distance between the stent edge and the vessel for the intraluminal images to a threshold distance and identifies which intraluminal images correspond to a distance exceeding the threshold. The processor circuit further outputs to a display a longitudinal view of the body lumen identifying locations along the stent at which the distance between the stent and the vessel wall exceeds the threshold distance.

The effective placement of endovascular devices is contingent upon accurately determining their location and shape. This implies that the precise positioning of the device in relation to proximal and distal landing targets, along with ensuring proper vessel wall apposition, are critical for the overall success of the procedure.

### SUMMARY OF THE INVENTION

There may thus be a need to provide a controller which assesses and tracks various parameters and features linked to device deployment during the deployment, and if any of these parameters exceed their threshold, the controller generates an output command. This output command may be employed to adjust the device's position both in terms of direction and velocity.

The object of the present invention is solved by the subject-matter of the independent claims; further embodiments are incorporated in the dependent claims. This invention offers the capability to extract device features from images and estimate the optimal device movement, ensuring its location, direction and/or shape align appropriately with the anatomy. Additionally, a controller can automate the positioning and deployment of devices when integrated with a robotic system.

The controller or data processor outlined in this disclosure has the potential to enhance standardization, increase precision and deployment accuracy, leading to improved care and reduced complications.

It should be noted that the following described aspects of the invention apply also for the device for assisting in placement of an endovascular device, for the corresponding method which may be implemented as computer program product, and for the non-transitory computer readable medium having stored such a computer program product.

In general, a device for assisting in placement of an endovascular device to be deployed for medical therapy comprises in accordance with the present disclosure a data processor configured to receive image data representing an anatomical region of interest including a vasculature, and at least a distal portion of a placement device together with the endovascular device located in the vasculature.

The received image data may be X-ray image data which may be generated, e.g., by an intra-operative C-arm based X-ray imaging device or the like. The anatomical region may be enhanced by a contrast agent in the X-ray image, i.e. the image data may represent an angiography image. Other devices for generating X-ray image data can be used.

It is noted that the endovascular device may be a stent-like device, a flow-diverter with a mesh-type structure, an intrasaccular device, an intra-cranial stent, but is not limited to such devices. The vasculature encompasses arterial and venous vessels as well as deformations of vessel walls like aneurisms. While a stent-like device may be placed in a vessel so as to open up a calcified portion thereof, a flow-diverter may be placed in a vessel so as to bridge or treat an aneurism, and a woven endobridge device may be placed in an aneurism so as to fill the same. In this disclosure, some aspects are described in the context of an intra-cranial flow diverter as an example providing an understanding of the invention.

The placement device, like a catheter, comprises a distal end, a proximal end, a longitudinal direction extending between the distal end and the proximal end, an outer sheath extending along the longitudinal direction, and an inner shaft extending along the longitudinal direction, wherein the outer sheath and/or the inner shaft are movable one to the other in the longitudinal direction of the placement device (i.e. the outer sheath is movable in the longitudinal direction of the placement device and/or the inner shaft is movable in the longitudinal direction of the placement device). The inner shaft may be a wire like a guidewire. An endovascular device, like those mentioned above, may be placed in the vicinity of the distal end of the placement device between the inner shaft and the outer sheath, for introduction of the endovascular device into the vasculature and to the intended placement site.

The endovascular device comprises a distal end, a proximal end, and an engagement feature for engagement at the distal end of the inner shaft of the placement device. It is noted that an engagement feature may be configured to block any movement of the endovascular device in a longitudinal direction when the endovascular device is compressed and placed between the inner shaft and the outer sheath of the placement device. The blocking of longitudinal movements is in particular of advantage for a partial deployment of the endovascular device, when the outer sheath is retracted to some extent but not fully retracted (and/or when the inner sheath is advanced to some extent but not fully advanced). The outer sheath is fully retracted (and/or the inner sheath is fully advanced) when the endovascular device can expand so that the engagement feature will disengage from the inner shaft of the placement device by a movement in a radial direction away from the inner shaft. In the context of this disclosure, retracting the outer sheath (and/or advancing the inner shaft) and allowing the endovascular device to expand is denoted as "unsheathing". It is noted that the engagement feature allows for pulling the endovascular device into the outer sheath of the placement device by either pulling the inner shaft proximally or by pushing the outer sheath distally. Bringing the endovascular device back into the outer sheath is denoted herein as "resheathing".

The data processor or controller is configured to interpret the image data and to determine instructions for positioning and deployment of the endovascular device while the endovascular device is in engagement with the inner shaft of the placement device. Interpreting the image data comprises at least one of an identification of the position of the endovascular device relative to the vasculature and of an identification of a vasculature wall apposition of the endovascular device. Such interpretation may be based on predetermined thresholds, wherein the thresholds may vary based on factors such as the type of the device, anatomical characteristics, device(s) distance to the distal target and the proximal target. In particular, the position of the endovascular device is identified in a longitudinal direction of the vasculature. Determining instructions for positioning and deployment comprises determining instructions for movement of the outer sheath and for movement of the inner shaft so that the endovascular device is positioned at a predetermined position in the vasculature and is deployed with a predetermined vasculature wall apposition.

The determined instructions for positioning and deployment of the endovascular device may include instructions of (i) moving the inner shaft together with the outer sheath by pushing the inner shaft and the outer sheath distally so that the position of the endovascular device is changed relative to the vasculature, (ii) moving the inner shaft together with the outer sheath by pulling the inner shaft and the outer sheath proximally so that the position of the endovascular device is changed relative to the vasculature, (iii) moving the inner shaft together with the outer sheath by pushing the inner shaft and the outer sheath distally so that the diameter of at least a portion of the endovascular device is increased for vasculature wall apposition, (iv) moving the outer sheath relative to the inner shaft so that the endovascular device is unsheathed, and (v) moving the outer sheath relative to the inner shaft so that the endovascular device is resheathed. It is noted that a combination of the mentioned movements is also envisaged. For example, the inner shaft may be pushed distally to a greater extent as the outer shaft, resulting in an improvement of the apposition at the vasculature wall and an unsheathing at the same time. This example reflects a combination of the movements mentioned above under (iii) and (iv). Other combinations may be applied for advantage. Summarizing, the inner shaft and the outer sheath can be moved independent from each other and any combination of movements of the inner shaft and the outer sheath may be appropriate so as to achieve both the intended position of the endovascular device relative to the longitudinal direction of the vasculature and the sufficient apposition at the vasculature wall. As will be understood by a skilled person, further elements may be provided working together with either or both of the inner shaft and the outer sheath so as to achieve the unsheathing or resheathing.

The data processor is configured to provide an output representing the determined instructions for positioning and deployment. The output may include a visualization of instructions for moving of the inner shaft and of the outer sheath on a display device so that a user may be guided when deploying an endovascular device in a vasculature. The display may be a monitor or a head mounted display allowing for an augmented visualization and/or a 3D view on the arrangement of the outer sheath and the inner shaft together with the instructions. For example, the output may indicate by arrows whether the outer sheath and/or the inner shaft shall be moved distally or proximally. Besides being visible, the output may also be textual or audible, but not limited to. The placement device may consequently comprise a handle with manual actuation elements engaging the proximal end of the inner shaft and engaging the proximal end of the outer sheath, so that the inner shaft and the outer sheath can be manually moved in accordance with the determined instructions.

Alternatively, and/or additionally, the output representing the determined instructions for positioning and deployment may include instructions executable by a robotic device performing the movements of the inner shaft and the movements of the outer sheath. In consequence, the robotic device may comprise a first feature engaging the proximal end of the inner shaft and a second feature engaging the proximal end of the outer sheath so that the inner shaft and the outer sheath are moved in accordance with the determined instructions for positioning and deployment of the endovascular device, when the robotic device executes the determined instructions. In other words, the data processor's decision unit transmits a movement signal and the robot controller directs the corresponding movements of the robot arms or end-effectors connected to surgical or endovascular devices.

The data processor may further incorporate additional auxiliary input related to tension or haptics. This supplementary information can be derived by either a dedicated tension estimator unit or by comparing the proximal movement of the device (e.g., recorded by robot encoders) with the movements observed at the distal end through visual imaging. The additional auxiliary input may also relate to the orientation of the proximal or distal segments of device(s).

The data processor may accommodate devices of known shapes such as shaped wires and catheters, steerable shapes (e.g., steerable catheters), and devices with tracked shapes (e.g., fiber optic real-shape sensing catheters). In the case of the latter, the tracking of the shape device's location can serve as auxiliary input to the data processor.

Further, the data processor may be configured to identify instances where successful deployment cannot be achieved prior to stent release. In response, the data processor may initiate actions such as full or partial resheathing or a complete change of the endovascular device.

The data processor may also examine both the distal and anticipated proximal segments of, e.g., a flow-diverter. Subsequently, it may issue pertinent commands to ensure a safe margin is maintained relative to the aneurysm. The data processor may verify whether the endovascular device is obstructing any perforators or side vessels. It may then adapt the output commands to prevent or rectify such obstructions.

The data processor may involve apposition measurement and control determined by the shape of the device and vasculature in 2D and/or 3D. The shape of the device or parent vessel can be established through segmentation, contour extraction, or key-point identification.

The data processor may be designed so that when the endovascular device is unsheathed until the point-of-no-return, it can detect this event and resheath the endovascular device if necessary. Additionally, if the controller is connected to a robot, it has the capability to temporarily halt the movement until the shape and location of the endovascular device are confirmed. Additionally, the controller may also evaluate the likelihood of successful endovascular device placement and only halts the movement at the point-of-no-return if the likelihood of successful endovascular device placement is lower than some predetermined threshold.

The data processor may support the placement of not only a single endovascular device but also multiple stacked or telescoped endovascular devices. The data processor may, e.g., process information including the shape of the first (or previous deployed) endovascular device, malapposition at the proximal end of the first (or previous deployed) endovascular device, shape of the anatomy near the proximal end, etc. to determine the distal end of the next endovascular device to be deployed such that the stacked deployment will improve the apposition at the proximal end of the first (or previous deployed) endovascular device. The data processor may also determine the size of the next endovascular device to be deployed. Furthermore, it facilitates ballooning the endovascular device or adjusting the movement of the micro-catheter both forward and backward, enhancing its own apposition as well as the apposition of the first (or previous deployed) endovascular device post-deployment.

The data processor may accommodate additional coordinated movements, including maneuvers designed for untangling or detaching processes.

The data processor may incorporate a controller system that is a model trained based on data. The model may take the form of a neural network or a classical machine learning model, trained using various techniques such as supervised, self-supervised, unsupervised learning, clustering, reinforcement learning, and more. Specifically, in a supervised training model, data from prior procedures can be utilized to train this controller model.

The data processor may perform complex apposition calculations using a digital twin or other simulation in order to determine optimal loading, unloading, unsheathing, resheathing, anchoring, etc.

Another aspect of the disclosure is a method for assisting in placement of an endovascular device. The method generally comprises the steps of receiving image data, interpreting the image data, determining instructions for positioning and deployment of the endovascular device, and providing an output representing the determined instructions.

As described above, the image data represent an anatomical region of interest including a vasculature, and at least a distal portion of a placement device together with the endovascular device located in the vasculature.

Interpreting the image data comprises at least one of an identification of the position of the endovascular device relative to the vasculature and of an identification of a vasculature wall apposition of the endovascular device, and determining instructions for positioning and deployment comprises determining instructions for movement of the outer sheath and for movement of the inner shaft, while the endovascular device is in engagement with the inner shaft, so that the endovascular device is positioned at a predetermined position in the vessel and is deployed with a predetermined vasculature wall apposition.

The determined instructions for positioning and deployment of the endovascular device may, in particular, include at least one of the instructions out of the group consisting of (i) moving the inner shaft together with the outer sheath by pushing the inner shaft and the outer sheath distally so that the position of the endovascular device is changed relative to the vasculature, (ii) moving the inner shaft together with the outer sheath by pulling the inner shaft and the outer sheath proximally so that the position of the endovascular device is changed relative to the vasculature, (iii) moving the inner shaft together with the outer sheath by pushing the inner shaft and the outer sheath distally so that the diameter of at least a portion of the endovascular device is increased for vasculature wall apposition, (iv) moving the inner shaft relative to the outer sheath so that the endovascular device is unsheathed, (v) moving the inner shaft relative to the outer sheath so that the endovascular device is resheathed. Any combination of those movements may also be applied for advantage.

As mentioned above, the movements may be performed manually or by a robotic device.

According to a further aspect of the disclosure, a computer program is provided implementing the steps of the method for assisting in placement of an endovascular device. In particular, the interpretation of received images and the determination of instructions suitable for achieving the reliable position within a vasculature and apposition at the vasculature wall may be implemented as computer program. It will be understood that such steps may be implemented utilizing artificial intelligence.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows a deployment controller with inputs and outputs.
Fig. 2 shows a micro-catheter introduced into a vessel.
Fig. 3 shows a micro-catheter together with a flow-diverter positioned in a vessel for deployment of the flow-diverter.
Fig. 4 shows an example of a flow-diverter with an insufficient apposition at the vessel wall.
Fig. 5 shows an example of a flow-diverter with inaccurate longitudinal localization within the vessel.
Fig. 6 shows an example of a flow-diverter properly deployed in a vessel.
Fig. 7 is a flow chart of a method of controlling a deployment of a flow-diverter.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

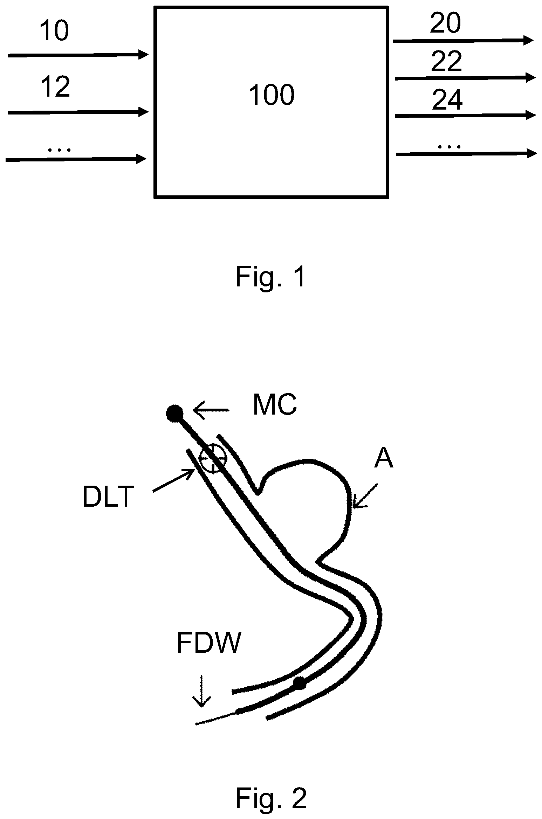

Fig. 1 schematically shows a deployment controller 100 designed to utilize imaging data acquired from stent-like devices for extracting device features. In Fig. 1, such input features are denoted as 10, 12, ... These features are then employed to identify optimal movements for deployment. The core element lies in the decision-making capabilities of the controller 100, which facilitates optimal movements of stent-like devices by considering imaging data, imaging parameters, and other auxiliary inputs. It is noted that the controller 100 may also be denoted as data processor or may comprise a data processor providing the functionality as described in this disclosure.

In the context of a flow-diverter (but may applicable to any other type of endovascular device deployable to perform a medical therapy of a vessel), the output movements encompass a range of actions, including but not limited to loading, unloading, unsheathing, resheathing, anchoring, and moving to the target. Such output movements are denoted in Fig. 1 as 20, 22, 24, ... The devices under consideration consist of, but are not restricted to, stents, flow-diverters, intrasaccular devices, and stent-retrievers. The execution of output commands may necessitate the movement of a single device (e.g., a flow-diverter) or the coordinated movement of multiple devices (e.g., a micro-catheter and a flow-diverter).

This system may utilize 2D imaging data, including fluoroscopy images, roadmap images, digitally subtracted images, etc., and/or 3D images such as 3D rotational angiographies or cone-beam CT scans as input 10, 12, .... Additional auxiliary inputs may also be incorporated, encompassing but not limited to device shapes, device characteristics information, pre- or intra-operative plans, anatomical information, and robotic data.

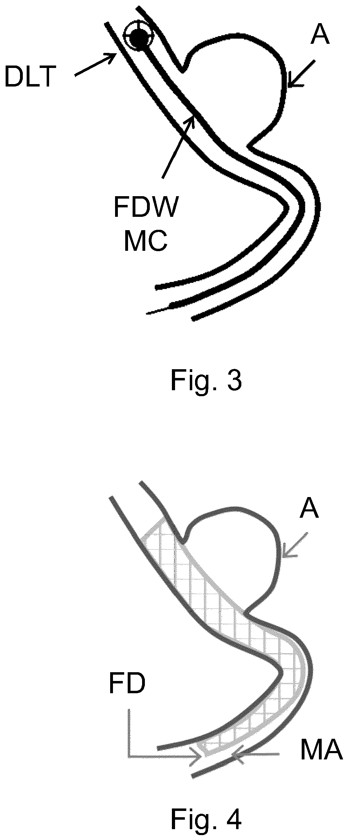

The deployment controller 100 can be assembled from either a single or multiple sub-control systems. The controller may be employed to navigate the devices to the treatment site. A device feature extraction module extracts crucial features, such as the distance between the tip of the micro-catheter and the delivery wire to a landing target. Using these features, appropriate commands are generated to maneuver the devices to the target. Exemplary commands include pull-back on the micro-catheter and push-forward on the delivery wire. The target location can be automatically determined by the feature extraction module or manually specified by the user. Fig. 2 and 3 show examples of micro-catheters and flow-diverter delivery wires moved so as to be at the distal landing target.

Fig. 2 is a schematic illustration of a vessel with an aneurysm A. Within the vessel, a micro catheter MC is placed and a flow-diverter delivery wire FDW is shown in a proximal section of the micro catheter MC. The distal end of the micro-catheter MC is shown with an enlarged head, although the micro-catheter MC may also be provided without such a head and, instead, with a continuous thickness at its distal end. The flow-diverter delivery wire FDW is configured to move like an inner shaft within the micro-catheter which may be considered as an outer sheath surrounding the inner flow-diverter delivery wire FDW. Further indicated in Fig. 2 is a designated landing target DLT. Fig. 2 can be seen as illustrating a state at the beginning of a procedure, i.e. before a placement of an endovascular device in a vessel.

Fig. 3 illustrates the vessel with the aneurysm A as in Fig. 2. Fig. 3 also shows the designated landing target DLT. As may be considered as a state initial to a deployment of a flow-diverter, both the flow-diverter delivery wire FDW and the micro-catheter MC are place with their respective distal end located at the designated landing target DLT.

The controller 100 may, during deployment of the flow-diverter, analyze the image and extracts features, such as the area and extent of malapposition. This module delineates the contour around the stent and assesses its overlap with the aneurysm's parent vessel. The uncovered area by the stent indicates the degree of malapposition. Whenever malapposition surpasses a threshold, the apposition controller generates commands to initiate system loading. Loading leads to stent expansion, enhancing vessel wall apposition. An exemplary loading command involves simultaneous push-forward on both the micro-catheter and the flow-diverter. When tension in the system builds up, the system can send command to unload the system by simultaneous pull-back on the micro-catheter and the flow-diverter.

Fig. 4 illustrates a situation with a flow-diverter FD being deployed within the vessel but with a malapposition MA at its proximal end portion.

Another sub-controller may ensure the flow-diverter opens at the designated landing target DLT. This controller analyzes the image during the unsheathing or deployment process of the flow-diverter. As an example, as the stent opens it may gently propel the flow-diverter stent upward, preventing it from dislodging from the designated landing target. Once the flow-diverter opens and anchors to the vessel walls, further upward push ceases to avoid excessive tension. In another example, during the anchoring phase, the distal end of the flow diverter FD may constantly move towards the desired distal landing target regardless of the current microcatheter movement. In another example, the controller may provide instructions to partially open the flow diverter mesh and move the delivery wire FDW and micro-catheter MC as a linked group until the flow diverter's distal end is in the desired position, then perform the deployment until anchoring is achieved. That is, throughout the deployment, it continually monitors the distal landing target. If the distance between the flow diverter distal end and the desired distal landing target exceeds a predetermined threshold, the controller generates commands to realign the flow-diverter with the distal landing target. The controller is also equipped to issue resheathing commands if the flow-diverter's location necessitates resheathing after anchoring has occurred.

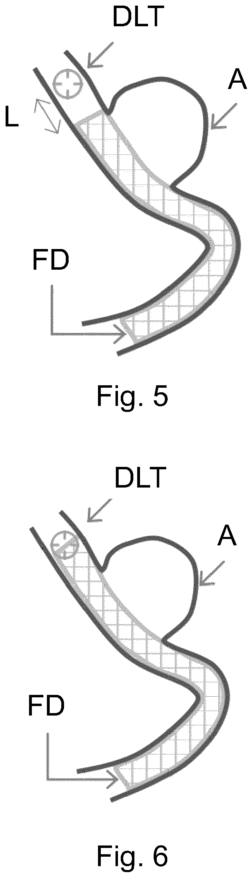

While Fig. 5 illustrates a flow-diverter FD positioned in a vessel with its distal end being away from the designated landing target DLT at a distance L, Fig. 6 show the flow-diverter FD with its distal end being accurately positioned at the designated landing target DLT. The positioning of the flow-diverter FD as in Fig. 6 ensures that blood flow through the vessel will be diverter away from the aneurysm A.

In the following, steps of an exemplary method are described for further understanding of the present disclosure. It will be understood that the steps described are major steps, wherein these major steps might be differentiated or divided into several sub-steps. Furthermore, there might be also sub-steps between these major steps. It will also be understood that only part of the whole method may constitute the invention, i.e. steps may be omitted.

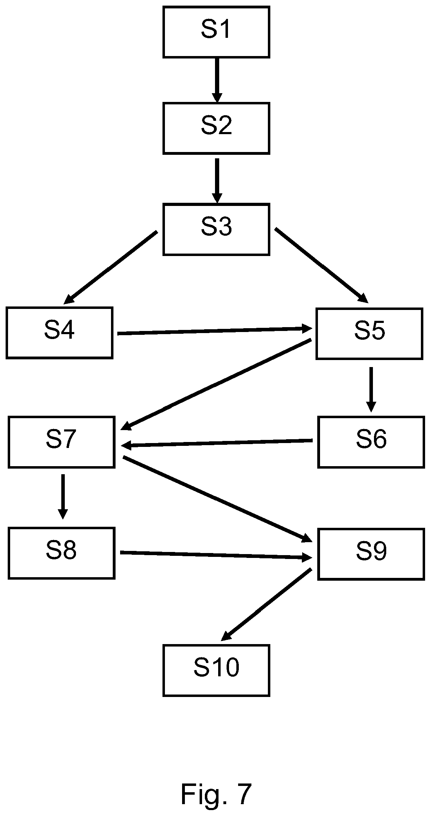

Steps for the deployment of a flow-diverter are schematically depicted in the flow-chart of Fig. 7. The initial decision made by the controller involves navigating the micro-catheter distal to the aneurysm (step S1). Subsequently, the tips of the micro-catheter and the flow-diverter are aligned with the distal landing target (step S2).

Following this, in one example, the micro-catheter may be retracted to unsheathe the flow-diverter (step S3). In other examples, depending on the type of flow diverter employed, other actions may be performed to unsheathe the flow diverter, such as pushing on the delivery wire. The controller, depending on whether the flow-diverter is open and securely anchored to the vessel wall, determines whether to move the flow-diverter or not. If the distal part of the flow-diverter is open, the system will automatically cease issuing push-forward commands for the flow-diverter (step S4).

While the micro-catheter undergoes unsheathing, the deployment controller consistently assesses vessel wall apposition by analyzing the device's shape and comparing it to the parent vessel (step S5). It identifies any gaps and determines the malapposition score based on the size of these gaps (step S6). If the malapposition measure surpasses the loading threshold, the controller will automatically issue the loading command to expand the flow-diverter (step S7). The deployment controller may also consider the proximity of the aneurysm neck or how the packing density will affect the desired proximal landing zone (step S8).

When the flow-diverter reaches the point of no return, the controller will generate output movements to fully deploy the flow-diverter (step S9). Subsequently, the controller will send commands to capture the wire and retract the entire device assembly (step S10).

Summarizing, this disclosure is about a computer-based controller of a deployment of a mesh-type device (e.g. stent, flow-diverter, and stent-retriever, balloon..) in a vessel (or another organic lumen) arranged to get dynamic monitoring data related to the positioning and deployment of said mesh-type device within the vessel, process said monitoring data to generate metrics representative of a state of the device deployment within the vessel, said metrics comprising direction of deployment (with respect to the directions of the adjacent vessel walls) and magnitude (or opening angle) of the mesh deployment, and generate an output signal if said metrics exceed a predetermined threshold, said threshold being predetermined so as to be representative of a too high level of risk for the vessel and/or for the deployment.

In a particular embodiment, this output signal is a command to be employed to robotically adjust the device's position both in terms of direction and magnitude. The output command may be movements encompassing a range of actions (e.g. loading, unloading, unsheathing, resheathing, anchoring, moving to the target...).

As an option, the controller is further arranged to get additional auxiliary inputs, encompassing but not limited to device shapes, device characteristics information, pre- or intra-operative plans, anatomical information, and robotic data, implement said process step based on the additional auxiliary inputs, further to the monitoring data.

Optionally said additional auxiliary input includes tension or haptics, and may be derived by either a dedicated tension estimator unit or by comparing the proximal movement of the device (e.g., recorded by robot encoders) with the movements observed at the distal end through visual imaging.

As an option said threshold can change based on factors such as the type of the device, anatomical characteristics, device(s) distance to the distal target, the point of no return, and the proximal target.

Furthermore, the controller may further be arranged to dynamically receive imaging data of said mesh-type device within the vessel, and dynamically extract said monitoring data related to the positioning and deployment of the device from the image features.

By this way, the invention lies in a decision-making capabilities of the controller, which facilitates optimal movements of a mesh-(or stent-)like device by optionally considering imaging data, imaging parameters, and other auxiliary inputs.

Optionally, the system comprises a user interface arranged to assign visual, textual, or auditory elements corresponding to the controller output signal.

The controller may further make use of a model trained on data, such as e.g. a neural network or a classical machine learning model, trained using various techniques such as supervised, self-supervised, unsupervised learning, clustering, reinforcement learning, and more. Specifically, in a supervised training model, data from prior procedures can be utilized to train this controller model.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application.

However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims. For instance the skilled person can clearly use the assistance of placement of a diverter to be deployed for treatment of an aneurysm as above-described in details, to other medical vascular therapies than aneurysm treatment, by e.g. using a stent-like deployment instead of a diverter deployment, used to perform a medical therapy of a blood vascular disease or damage (e.g. local aneurysm, narrowed vessels due to occlusion due to clots, calcification..., vascular lesion ...).

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device for assisting in placement of an endovascular device to be deployed for medical therapy, the device comprising a data processor configured to receive image data,
wherein the image data represent an anatomical region of interest including a vasculature, and at least a distal portion of a flexible elongated placement device together with the endovascular device located in the vasculature,
wherein the placement device comprises a distal end, a proximal end, a longitudinal direction extending between the distal end and the proximal end, an outer sheath extending along the longitudinal direction, and an inner shaft extending along the longitudinal direction, wherein the outer sheath and/or the inner shaft are movable one to the other in the longitudinal direction of the placement device,
wherein the endovascular device comprises a distal end, a proximal end, and an engagement feature for engagement at the distal end of the inner shaft of the placement device,
wherein the data processor is configured to interpret the image data and to determine instructions for positioning and deployment of the endovascular device while the endovascular device is in engagement with the inner shaft of the placement device,
wherein interpreting the image data comprises at least one of an identification of the position of the endovascular device relative to the vasculature and of an identification of a vasculature wall apposition of the endovascular device, and wherein determining instructions for positioning and deployment comprises determining instructions for movement of the outer sheath and for movement of the inner shaft so that the endovascular device is positioned at a predetermined position in the vasculature and is deployed with a predetermined vasculature wall apposition,
wherein the data processor is further configured to provide an output representing the determined instructions for positioning and deployment.

2. The device of claim 1, wherein the determined instructions for positioning and deployment of the endovascular device include at least one of the instructions out of the group consisting of (i) moving the inner shaft together with the outer sheath by pushing the inner shaft and the outer sheath distally so that the position of the endovascular device is changed relative to the vasculature, (ii) moving the inner shaft together with the outer sheath by pulling the inner shaft and the outer sheath proximally so that the position of the endovascular device is changed relative to the vasculature, (iii) moving the inner shaft together with the outer sheath by pushing the inner shaft and the outer sheath distally so that the diameter of at least a portion of the endovascular device is increased for vasculature wall apposition, (iv) moving the outer sheath and the inner shaft relative to each other so that the endovascular device is unsheathed, (v) moving the outer sheath and the inner shaft relative to each other so that the endovascular device is resheathed.

3. The device of any one of claims 1 and 2, wherein the output representing the determined instructions for positioning and deployment includes a visualization of instructions for moving of the inner shaft and of the outer sheath on a display device.

4. The device of claim 3, further comprising a display, wherein the placement device comprises a handle engaging the proximal end of the inner shaft and engaging the proximal end of the outer sheath, so that the inner shaft and the outer sheath can be manually moved in accordance with the visualized instructions.

5. The device of any one of claims 1 to 4, wherein the output representing the determined instructions for positioning and deployment includes instructions executable by a robotic device performing the movements of the inner shaft and/or the movements of the outer sheath.

6. The device of claim 5, further comprising a robotic device, wherein the robotic device comprises a first feature engaging the proximal end of the inner shaft and/or a second feature engaging the proximal end of the outer sheath so that the inner shaft and/or the outer sheath are moved in accordance with the determined instructions for positioning and deployment of the endovascular device, when the robotic device executes the determined instructions.

7. The device of any one of claims 1 to 6, wherein the endovascular device is one out of the group consisting of a stent-like device, a flow-diverter with a mesh-type structure, a woven endobridge device, and an intra-cranial stent.

8. The device of any one of claims 1 to 7, wherein the image data comprises X-ray image data.

9. The device of any one of claims 1 to 6, wherein the endovascular device is adapted to treat an aneurism.

10. The device of claim 9, wherein the endovascular device is a flow-diverter configured to be placed in an intra-cranial aneurism parent vessel.

11. A method for assisting in placement of an endovascular device, the method comprising the steps of
receiving image data,
interpreting the image data,
determining instructions for positioning and deployment of the endovascular device and providing an output representing the determined instructions,
wherein the image data represent an anatomical region of interest including a vasculature, and at least a distal portion of a flexible elongated placement device together with the endovascular device located in the vasculature,
wherein the placement device comprises a distal end, a proximal end, a longitudinal direction extending between the distal end and the proximal end, an outer sheath extending along the longitudinal direction, and an inner shaft extending along the longitudinal direction, wherein the outer sheath and/or the inner shaft are movable one to the other in the longitudinal direction of the placement device and wherein,
wherein the endovascular device comprises a distal end, a proximal end, and an engagement feature for engagement at the distal end of the inner shaft of the placement device,
wherein interpreting the image data comprises at least one of an identification of the position of the endovascular device relative to the vasculature and of an identification of a vasculature wall apposition of the endovascular device, and
wherein determining instructions for positioning and deployment comprises determining instructions for movement of the outer sheath and for movement of the inner shaft, while the endovascular device is in engagement with the inner shaft, so that the endovascular device is positioned at a predetermined position in the vessel and is deployed with a predetermined vasculature wall apposition.

12. The method of claim 11, wherein the determined instructions for positioning and deployment of the endovascular device include at least one of the instructions out of the group consisting of (i) moving the inner shaft together with the outer sheath by pushing the inner shaft and the outer sheath distally so that the position of the endovascular device is changed relative to the vasculature, (ii) moving the inner shaft together with the outer sheath by pulling the inner shaft and the outer sheath proximally so that the position of the endovascular device is changed relative to the vasculature, (iii) moving the inner shaft together with the outer sheath by pushing the inner shaft and the outer sheath distally so that the diameter of at least a portion of the endovascular device is increased for vasculature wall apposition, (iv) moving the inner shaft and the outer sheath relative to each other so that the endovascular device is unsheathed, (v) moving the inner shaft and the outer sheath relative to each other so that the endovascular device is resheathed.

13. The method of claim 12, wherein the movements are performed by a robotic device.

14. A computer program product comprising processor-executable instructions which when executed on the data processor of the device according to any one of claims 1 to 10, cause the device to carry out the method according to any one of claims 11 to 13.

15. A non-transitory computer readable medium having stored the computer program of claim 14.
